# EUROPEAN PATENT APPLICATION

(11) **EP 3 928 684 A1**
(43) Date of publication of application: **29.12.2021**
(21) Application number: 20182416.6
(22) Date of filing: 26.06.2020
(51) Int. Cl.: A61B 5/00, A61B 5/0205, A61B 5/026, A61B 5/08, A61B 5/11, A61B 5/113, A61B 5/055

(54) **CONTACTLESS TEMPERATURE-BASED MONITORING OF A PATIENT**

(71) Applicant: Koninklijke Philips N.V., 5656 AG Eindhoven (NL)
(72) Inventor: KRUEGER, Sascha, 5656 AE Eindhoven (NL)
(74) Representative: Philips Intellectual Property & Standards

(57) **Abstract**

According to the invention, a method for detecting at least one physiological signal (1) of a patient (7), wherein the method comprises the following method steps: monitoring at least a subsection (2) of a patient's surface (4) with a thermal camera (5) which generates consecutive video frames with multiple pixels (6) of the monitored subsection (2), wherein the subsection (2) of a patient's surface (4) includes at least a part of the mouth and/or nose area of the patient (7) as a region of interest (3); generating time-resolved temperature values of at least one pixel (8) of the region of interest (3); and generating a cardiac signal (9) as the physiological signal (1) based on the generated time-resolved temperature values. In this way, a possibility is provided for contactless temperature-based monitoring of a patient in an easy and cost-efficient way.

## Description

### FIELD OF THE INVENTION

The invention relates to the field of detecting breathing motion and cardiac motion of a patient and in particular to the detection of breathing motion and cardiac motion during medical imaging examinations or scan acquisitions.

### BACKGROUND OF THE INVENTION

The detection of breathing motion and cardiac motion of a patient has conventionally been done using sensors applied to the patient. Physiology signals for breathing and pulse may be provided by the analysis of a live video stream of a patient either in RGB or IR mode. For detection of breathing motion, visibility of the motion of the target body part such as the chest or the upper abdomen is required. In typical medical imaging scenarios coils, accessories, blankets or support devices may obstruct the view from a particular fixed view angle thus limiting the applicability of this technique. For non-obstructive remote cardiac physiology signal detection, also video data can be used to detect the color changes in the skin due to the pulsatile flow. The principle of measurement is then based on the pulse plethysmography principle. The signal strength may be reduced by different aspects, i.e. by the visibility of the skin due to top or facial hair or by the physiologic delay which may vary from patient to patient or for a single patient even over time. In general, video-based pulse plethysmography works well with a multi-spectral detector so that absorption and reflection properties of the skin can be self-calibrated for each video-frame. A preferred setup for such a technique is based on a visible RGB camera employing visible white light.

In Hu M, Zhai G, Li D, Fan Y, Duan H, Zhu W, et al. "Combination of near-infrared and thermal imaging techniques for the remote and simultaneous measurements of breathing and heart rates under sleep situation" in PLoS ONE 13(1) 2018 a far-infrared imager and an infrared camera equipped with IR-Cut lens and an infrared lighting array are described. The heart rate is detected by the infrared imaging system based on the absorption changes of the skin due to the blood flow in order to describe the quality of sleep.

### SUMMARY OF THE INVENTION

It is an object of the invention to provide a monitoring method and a monitoring system for detecting at least cardiac motion of a patient in an easy, versatile and reliable way, especially without the need of irradiating additional light.

According to the invention, this object is addressed by the subject matter of the independent claims. Preferred embodiments of the invention are described in the sub claims.

Therefore, according to the invention, a method for detecting at least one physiological signal of a patient is provided, wherein the method comprises the following method steps: monitoring at least a subsection of a patient's surface with a thermal camera which generates consecutive video frames with multiple pixels of the monitored subsection, wherein the subsection of a patient's surface includes at least a part of the mouth and/or nose area of the patient as a region of interest; generating time-resolved temperature values of at least one pixel of the region of interest; and generating a cardiac signal as the physiological signal based on the generated time-resolved temperature values.

If a cardiac signal is mentioned, a signal generated from the vibration wave following the contraction of the heart muscle in the chest is meant. The heart rate shown by the cardiac signal is the speed of the heartbeat which is typically measured by the number of heart beats per minute.

The method is adapted for generating a physiological signal based on temperature values of breathing air flow. That is why the region of interest includes the mouth and nose area or only the mouth or only the nose area.

According to a preferred embodiment of the invention, the method further comprises the method step of generating a respiration signal as an additional physiological signal based on the generated time-resolved temperature values.

If a respiration signal is mentioned, a signal generated from the inhalation and exhalation process of a patient's breathing is meant. The breathing rate is the number of breaths a person takes per time, e.g. per minute. During generating a respiration signal based on the generated temperature values, a low pixel temperature relative to the reference temperature indicates inhalation and a high pixel temperature relative to the reference temperature indicates exhalation.

According to a preferred embodiment of the invention, the method further comprises the method step of triggering and/or gating a scan acquisition based on the at least one physiological signal, wherein the scan acquisition is carried out during medical imaging examination with a medical imaging device and/or during medical therapy. Breathing and heart motions cause uncertainties during medical imaging examinations and/or during medical therapies, because organs, or ROIs in general, are moving during scan acquisition. The image data acquisition is triggered in dependence on the breathing motion and/or the cardiac motion, so that the uncertainties because of moving ROIs during scan acquisition are reduced. A medical imaging device can be for example MRI, CT, PET and a medical therapy can be for example radiotherapy.

A physiological signal is generated based on temperature values of breathing air flow. For generating temperature values of breathing air flow, the region of interest includes the mouth and nose area or only the mouth or only the nose area According to a preferred embodiment of the invention, the at least one pixel is from the pixels covering the patient's nostrils.

One advantage resides in providing a method, wherein in the step of generating time-resolved temperature values of at least one pixel of the region of interest one single pixel is used. Hence, it is not necessary to generate time-resolved values of all pixels of the region of interest. Time-resolved temperature values of only one single pixel may be sufficient to generate a physiological signal.

Further, according to the invention, a monitoring system for detecting at least one physiological signal of a patient is provided. The monitoring system comprises a thermal camera which is adapted for monitoring at least a subsection of a patient's surface. The thermal camera generates consecutive video frames with multiple pixels of the monitored subsection. The subsection of the patient's surface includes at least a part of the mouth and/or nose area of the patient as a region of interest. The monitoring system further comprises a signal processing unit which is adapted for generating time-resolved temperature values of at least one pixel of the region of interest and for generating a cardiac signal as the physiological signal based on the generated time-resolved temperature values.

According to a preferred embodiment of the invention, the monitoring system comprises a patient support which is adapted for holding the patient in such a way that the subsection of the patient's surface which may be monitored by the thermal camera comprises at least a part of the mouth and/or nose area of the patient. The patient support may be designed as a patient table that may be movable in all three spatial directions so that the positioning of the patient can be done very precisely. Additionally, also face and/or body tracking devices and algorithms can be used to further confine the region of interest. The camera itself may be such a device and known computer vision algorithms could be applied to determine e.g. facial or head area.

According to a preferred embodiment of the invention, the signal processing unit is adapted for generating a respiration signal based on the generated time-resolved temperature values as an additional physiological signal. For generating time-resolved temperature values, preferably a frequency range for the pulse is defined. The range may be for example between 40 and 300 beats per minute. For generating a second physiologically signal, preferably a range for respiration is defined. The range may be for example 10 to 20 breathing cycles per minute. By defining ranges, the contributions by band-passing may be separated. According to another preferred embodiment it is made use of the spatial shifting of the head due to the respiration which is larger than the pulse-induced motion. This shifting may be measured in order to get a clean respiration curve and to use its frequency to separate it in the thermal signal.

Further, according to the invention, a non-transitory computer-readable medium is provided which comprises instructions stored thereon, that when executed on a processor, induce a monitoring system with a thermal camera to perform the method a method for detecting at least one physiological signal of a patient.

### BRIEF DESCRIPTION OF THE DRAWINGS

These and other aspects of the invention will be apparent from and elucidated with reference to the embodiments described hereinafter. Such an embodiment does not necessarily represent the full scope of the invention, however, and reference is made therefore to the claims and herein for interpreting the scope of the invention.

In the drawings:
Fig. 1 schematically depicts a scheme of a method according to a preferred embodiment of the invention;
Fig. 2 schematically depicts a second scheme of a method according to a preferred embodiment of the invention;
Fig. 3 schematically depicts a monitoring system according to a preferred embodiment of the invention; and
Fig. 4 schematically depicts a thermal camera and a ROI of a monitoring system according to a preferred embodiment of the invention.

### DETAILED DESCRIPTION OF EMBODIMENTS

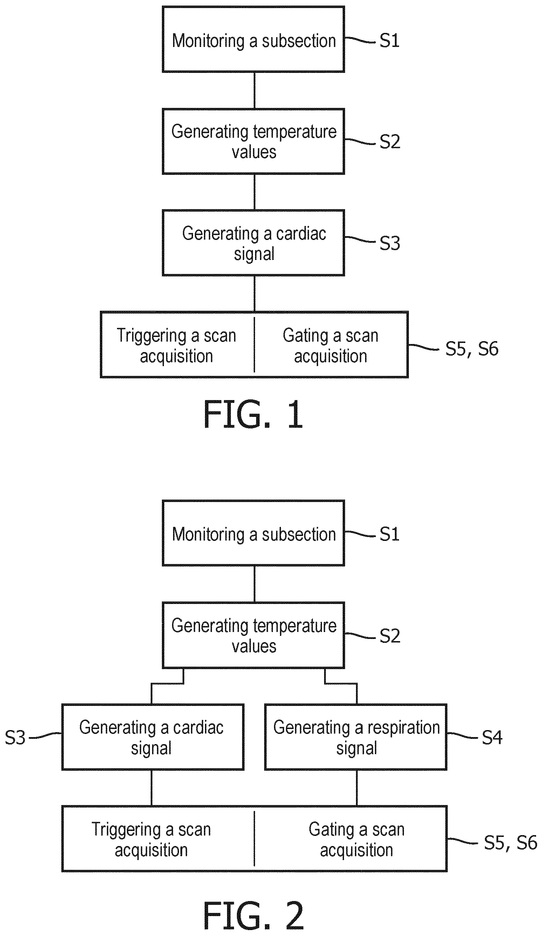

Fig. 1 schematically depicts a scheme of a method according to a preferred embodiment of the invention. The first step S1 is to monitor at least a subsection 2 of a patient's surface 4 with a thermal camera 5. The thermal camera 5 generates consecutive video frames with multiple pixels 6 of the monitored subsection 2. In order to generate a physiological signal based on temperature values of breathing air flow, the subsection 2 of a patient's surface 4 includes at least a part of the mouth and/or nose area of the patient 7 as a region of interest 3. The second step S2 is to generate these time-resolved temperature values of at least one pixel 8 of the region of interest 3. The third step S3 is to generate a cardiac signal 9 as the physiological signal 1 based on the generated time-resolved temperature values. The generated cardiac signal 9 can be used for the fifth step S5 and/or for the sixth step S6. The fifth step S5 is to trigger and the sixth step S6 is to gate a scan acquisition based on the at least one physiological signal 1.

A scan acquisition is carried out during medical imaging examination with a medical imaging device 11 and/or during medical therapy. Hence, it is possible to reduce uncertainties because of heart motions during medical imaging examinations and/or during medical therapies. Some regions are moving during scan acquisition because of the beating heart. The image data acquisition may be gated in dependence on the cardiac signal 9, so that the cardiac motion can be traced on medical images. Another opportunity is to trigger the image data acquisition in dependence on the cardiac signal 9, so that the image data acquisition is triggered every time the heart is in the same position. In Fig. 1 the medical imaging device 11 is a MRI system, a CT or a linear accelerator.

Fig. 2 schematically depicts a second scheme of a method according to a preferred embodiment of the invention. After the second step S2, thus after generating time-resolved temperature values of at least one pixel 8 of the region of interest 3, it is not only possible to generate a cardiac signal, as it is described in the third step S3 in Fig. 1, it is also possible to generate a respiration signal 10 as an additional physiological signal 1 based on the generated time-resolved temperature values. Generating a respiration signal 10 is the fourth step S4. Based on the cardiac signal 9 and/or the respiration signal 10 the image data acquisition may be triggered S5 and/or gated S6.

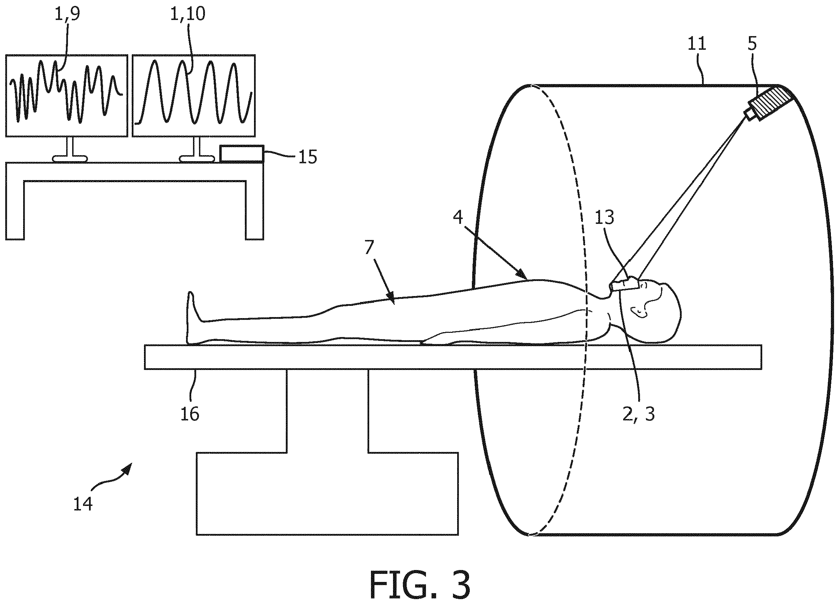

Fig. 3 schematically depicts a monitoring system 14 according to a preferred embodiment of the invention. A situation during image data acquisition is shown. A patient 7 is lying on a patient table 16. The table is movable in all three dimensions so that the region of interest 3 may be positioned precisely. The region of interest 3 is a part of a subsection 2 of the patient 7 and includes the patient's nostrils 13. A thermal camera 5 is attached to the medical imaging device 11. In Fig. 3 the medical imaging device 11 is a MRI. The thermal camera 5 scans the patient's surface 4, especially the precisely positioned region of interest 3. The thermal camera 5 is adapted to generate time-resolved temperature-values that are processed by a signal processing unit 15. The signal processing unit 15 generates two physiological signals based on the time-resolved temperature-values: a cardiac signal 9 and a respiration signal 10 that are visualized.

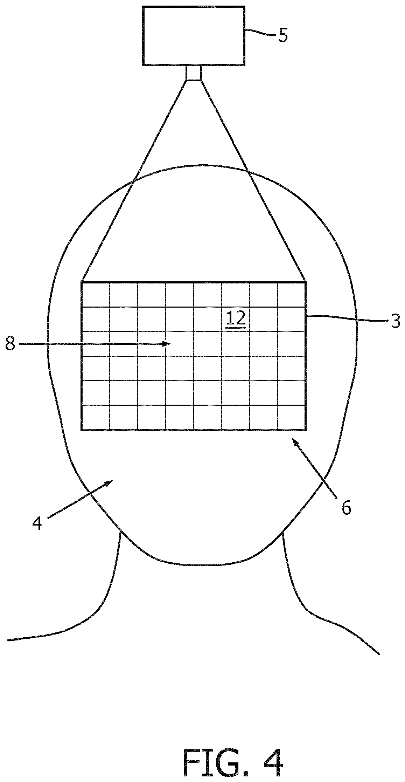

Fig. 4 schematically depicts a thermal camera 5 and a region of interest 3 of a monitoring system according to a preferred embodiment of the invention. The thermal camera 5 scans the patient's surface 4 and generates consecutive video frames with multiple pixels 6 of the region of interest 3. For the sake of clarity, the patient's nostrils 13 are not shown in Fig. 4, but they are part of the region of interest 3. Time-resolved temperature values of at least one pixel 8 of the region of interest 3 are generated. Especially according to the invention, it is possible to generate time-resolved temperature values of at least one pixel 8 of the region of interest 3 with only one single pixel 12 used.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope. Further, for the sake of cleamess, not all elements in the drawings may have been supplied with reference signs.

### REFERENCE SYMBOL LIST

| | |
|---|---|
| monitoring a subsection | S1 |
| generating temperature values | S2 |
| generating a cardiac signal | S3 |
| generating respiration signal | S4 |
| triggering a scan acquisition | S5 |
| gating a scan acquisition | S6 |
| physiological signal | 1 |
| subsection | 2 |
| region of interest | 3 |
| patient's surface | 4 |
| thermal camera | 5 |
| multiple pixels | 6 |
| patient | 7 |
| pixel | 8 |
| cardiac signal | 9 |
| respiration signal | 10 |
| medical imaging device | 11 |
| one single pixel | 12 |
| nostrils | 13 |
| monitoring system | 14 |
| signal processing unit | 15 |
| patient table | 16 |

## Claims

1. A method for detecting at least one physiological signal (1) of a patient (7), wherein the method comprises the following method steps:
monitoring at least a subsection (2) of a patient's surface (4) with a thermal camera (5) which generates consecutive video frames with multiple pixels (6) of the monitored subsection (2), wherein the subsection (2) of a patient's surface (4) includes at least a part of the mouth and/or nose area of the patient (7) as a region of interest (3);
generating time-resolved temperature values of at least one pixel (8) of the region of interest (3); and
generating a cardiac signal (9) as the physiological signal (1) based on the generated time-resolved temperature values.

2. The method of claim 1, wherein the method comprises the method step of
generating a respiration signal (10) as an additional physiological signal (1) based on the generated time-resolved temperature values.

3. The method of claim 1 or 2, wherein the method comprises the method step of
triggering and/or gating a scan acquisition based on the at least one physiological signal (1), wherein the scan acquisition is carried out during medical imaging examination with a medical imaging device (11) and/or during medical therapy.

4. The method of any of the previous claims, wherein the at least one pixel (12) is from the pixels (6) covering the patient's nostrils (13).

5. The method of any of the previous claims, wherein in the step of generating time-resolved temperature values of at least one pixel (8) of the region of interest (3) one single pixel (12) is used.

6. A monitoring system (14) for detecting at least one physiological signal (1) of a patient (7), wherein the monitoring system (14) comprises
a thermal camera (5) which is adapted for monitoring at least a subsection (2) of a patient's surface (4) with a thermal camera (5) which generates consecutive video frames with multiple pixels (6) of the monitored subsection (2), wherein the subsection (2) of the patient's surface (4) includes at least a part of the mouth and/or nose area of the patient (7) as a region of interest (3); and
a signal processing unit (15) which is adapted for generating time-resolved temperature values of at least one pixel (8) of the region of interest (3) and for generating a cardiac signal (9) as the physiological signal (1) based on the generated time-resolved temperature values.

7. The monitoring system (14) of claim 6, wherein the monitoring system (14) comprises a patient support (16) which is adapted for holding the patient (7) in such a way that the subsection (2) of the patient's surface (4) which may be monitored by the thermal camera (5) comprises at least a part of the mouth and/or nose area of the patient (7).

8. The monitoring system (14) of claim 6 or 7, wherein the signal processing unit (15) is adapted for generating a respiration signal (10) based on the generated time-resolved temperature values as an additional physiological signal (1).

9. A non-transitory computer-readable medium, comprising instructions stored thereon, that when executed on a processor, induce a monitoring system (14) with a thermal camera (5) to perform the method according to any of claims 1-5.
